# EUROPEAN PATENT APPLICATION

(11) **EP 1 055 733 A1**
(43) Date of publication of application: **29.11.2000**
(21) Application number: 99110122.1
(22) Date of filing: 25.05.1999
(51) Int. Cl.: C12Q 1/37

(54) **FmhB-protein and modulators of this protein**

(71) Applicant: Bayer Aktiengesellschaft, 51368 Leverkusen (DE)
(72) Inventor: Berger-Bächi, Brigitte, Prof. Dr., 8121 Benglen (CH); Tschierske, Martin, Dr., 8051 Zürich (CH); Rohrer, Susanne, 8051 Zürich (CH); Ehlert, Kerstin, Dr., 42115 Wuppertal (DE)

(57) **Abstract**

The factor catalysing the first step in the synthesis of the characteristic pentaglycine interpeptide in the *S. aureus* peptidoglycan was found to be encoded by the essential gene *fmhB*. It was found that repression of *fmhB* reduces the bacterial growth-rate and triggers a drastic accumulation of un-crosslinked, un-modified muropeptide precursos hereby destabilizing cell wall integrity.

Any shortening of said pentaglycine interpeptide reduces or even abolishes methicillin resistance as occurred after *fmhB* repression. Because of its key role FmhB is a general target for novel antimicrobial agents that could control the threat of emerging multiresistant *S. aureus* and any other microorganisms containing interpeptide bridges in their cell walls and requiring a fully functional FmhB or its homologs for biosynthesis of said interpeptide bridges.

Such microorganisms are all those containing orthologous genes to *fmhB*, for instance other Staphylococci, Streptococci, Enterococci, and other species.

SEQUENCE LISTING

<110> Bayer AG

<120> FmhB - Protein and modulators of this protein.

<130> LeA 33779

<140>
<141>

<160> 7

<170> PatentIn Ver. 2.0

<210> 1
<211> 1669
<212> DNA
<213> Staphylococcus aureus

<400> 1
cgataacatt aaatttaaca cgaaacatag atataaaatg attcacaatt aaaatgggta 60
aatttgaact tgctaaacta ttaattggag catggacatt tcaaaaataa gagttcaaat 120
cttacacaag ctctgaatcg acactataag atacaaactg tataattaaa ggtattgtta 180
aatagaagga gatatcataa atcatggaaa agatgcatat cactaatcag gaacatgacg 240
catttgttaa atctcaccca aatggagatt tattacaatt aacgaaatgg gcagaaacaa 300
agaaattaac tggatggtac gcgcgaagaa tcgctgtagg tcgtgacggt gaagttcagg 360
gtgttgcgca attacttttt aaaaaagtac ctaaattacc gtatacgcta tgttatattt 420
cacgtggttt tgttgttgat tatagtaata aagaagcgtt aaatgcattg ttagacagtg 480
caaaagaaat tgctaaagct gagaaagcgt atgcaattaa aatcgatcct gatgttgaag 540
ttgataaagg tacagatgct ttgcaaaatt tgaaagcgct tggttttaaa cataaaggat 600
ttaaagaagg tttatcaaaa gactacatcc aaccacgtat gactatgatt acaccaattg 660
ataaaaatga tgatgagtta ttaaatagtt ttgaacgccg aaatcgttca aaagtgcgct 720
tggctttaaa gcgaggtacg acagtagaac gatctgatag agaaggttta aaaacatttg 780
ctgaattaat gaaaatcact ggggaacgcg atggcttctt aacgcgtgat attagttact 840
ttgaaaatat ttatgatgcg ttgcatgaag atggagatgc tgaactattt ttagtaaagt 900
tggacccaaa agaaaatata gcgaaagtaa atcaagaatt gaatgaactt catgccgaaa 960
tagctaaatg gcagcagaag atggaaacat ctgaaaagca agctaaaaaa gcgcaaaata 1020
tgattaatga tgcgcaaaat aaaattgcta aaaatgaaga tttaaaacga gacctagaag 1080
ctttagaaaa ggaacatcct gaaggtattt atctttctgg tgcactatta atgtttgctg 1140
gctcaaaatc atattactta tatggtgcgt cttctaatga atttagagat tttttaccaa 1200
atcaccatat gcagtatacg atgatgaagt atgcacgtga acatggtgca acaacttacg 1260

atttcggtgg tacagataat gatccagata aagactctga acattatggc ttatgggcat 1320
ttaaaaaagt gtggggaaca tacttaagtg aaaagattgg tgaatttgat tatatattga 1380
atcagccatt gtaccaatta attgagcaag ttaaaccgcg tttaacaaaa gctaaattta 1440
aaatatctcg taaattaaaa cgaaaataga ttaacgactg aaatctgaac gctcataaga 1500
ctgtcatttg cgttcagatt tttttacaca atatagaatg gttgagtaaa atatttttga 1560
atatagtgaa agagggggaa gtactgtgat aaaaaagcta ttacaatttt ctttggggaa 1620
taagtttgct atctttttaa tggttgtttt agttgtcttg ggcggtgta 1669

<210> 2
<211> 31
<212> DNA
<213> Staphylococcus aureus

<400> 2
gcggatccat tgttaaatag aaggagatat c 31

<210> 3
<211> 28
<212> DNA
<213> Staphylococcus aureus

<400> 3
gcggtacccc cagtgatttt cattaatt 28

<210> 4
<211> 24
<212> DNA
<213> Staphylococcus aureus

<400> 4
aaatgaacaa tgtgctatat tacc 24

<210> 5
<211> 21
<212> DNA
<213> Staphylococcus aureus

<400> 5
gccagcaaac attaatagtg c 21

<210> 6
<211> 29

<212> DNA
<213> Staphylococcus aureus

<400> 6
gcgaattctt acacaagctc tgaatcgac 29

<210> 7
<211> 30
<212> DNA
<213> Staphylococcus aureus

<400> 7
gcctgcagat ttagctattt cggcatgaag 30

## Description

### Technical Field

The bacterial cell wall plays an important role in infection and pathogenicity. Because of the uniqueness of the peptidoglycan structure and assembly it is one of the preferred targets of antibiotics. However, evolution always brings forth bacterial strains which prove to be resistant to any so far existent cures. This raises steady need to search for new targets which are pivotal for bacterial survival in order to hit a broad variety of bacterial genera.

Starting off from investigations regarding methicillin resistance in *S. aureus* we found that the addition of the first glycine residue in staphylococcal interpeptide bridge biosynthesis depends on the proper functioning of the FmhB protein of *S. aureus*. Hence FmhB is an interesting target for antibacterial treatment and the current invention relates to the use of said gene, its products, and compounds modulating the function or in particular, which inhibit the function of said gene and its products as new means for antimicrobial treatment of *S. aureus* and other microorganisms containing that gene.

Further the knowledge derived from the teaching of this invention enables to set up assays suitable to determine the activity of FmhB and/or their homologues under in vitro and in vivo conditions. Such assays for example are assays wherein the addition of the first glycine to a murein precursor is determined and wherein such a murein precursor is an unmodified murein precursor.

Since any shortening of the glycine interpeptide bridges causes a drastic change in the susceptibility towards lysostaphin, this fact renders this enzyme ideal for use in assays for the detection of structural derivations in the cell wall of bacteria that had been submitted to a modulator of the function of FemAB or FmhB.

The scope of the invention in particular comprises the use of the FmhB protein and homologs thereof in the screening for antimicrobial compounds, and that is precisely why compounds capable to bind to FmhB, compounds capable to modulate the activity of FmhB, and compounds capable to inhibit the activity of FmhB lie within the scope of the invention.

Within the scope of the invention lies further the use of lysostaphin as a tool for the detection of the activities of FmhB and FemAB and their homologs in cell based screening assays.

Because of the biological importance that is shown for FmhB it can be concluded that any pharmaceutical composition comprising a compound capable to either bind to FmhB, or modulate the activity of Fmhb, or inhibit the activity of FmhB, falls under the scope of this invention. With regard to the ability of FmhB to modulate or abolish bacterial resistance against antibiotic cures and especially such cures where said antibiotic is a β-lactam antibiotic, to be consistent, it is concluded that any pharmaceutical composition or combination comprising a compound acting towards FmhB in any of the above mentioned ways in combination with an antibiotic falls under the scope of this invention.

It is known that *S. aureus* peptidoglycan consists of linear sugar chains of alternating units of N-acetylglucosamine and N-acetylmuramic acid substituted with the pentapeptide L-alanyl-D-isoglutaminyl-L-lysyl-D-alanyl-D-alanine. Characteristic for *S. aureus* is the pentaglycyl side-chain which connects the ε-amino group of L-lysine of the stem peptide to the penultimate D-alanyl of a neighbour.

The flexible pentaglycyl interpeptide allows a peptidoglycan crosslinking degree of up to 90%, thus contributing substantially to cell wall stability ¹⁾ and references cited therein. In addition, this pentaglycyl chain acts as recipient for staphylococcal surface proteins which are covalently anchored to it by a transpeptidase-like reaction. Such surface proteins play an important role in adhesion and pathogenicity by interacting with the hosts matrix proteins.

It is further known that an additional low affinity penicillin-binding protein ²⁾,PBP2 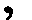 , absent in susceptible *S. aureus* strains is a pre-requisite for full expression of resistance towards methicillin and other β-lactams in MRSA (MRSA Methicillin Resistant *S. aureus*) strains.

However, besides a fully functional PBP2 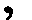 additional factors present in susceptible as well as resistant strains have been shown to be necessary for phenotypic methicillin resistance ¹⁾. These factors are the so called *fem*-factors. (*fem* factor essential for methicillin resistance). Up to now several *fem*-factors which are somehow involved in peptidoglycan biosynthesis, have been described.

Among these factors the FemAB proteins have been shown to be involved in the addition of the glycine-residues two to five of the staphylococcal pentaglycine interpeptide bridge. Inactivation of said FemAB proteins alone not only causes reduction of methicillin resistance and hypersusceptibility to other antibiotics but also give rise to a drastically reduced growth rate and impairment of cell wall stability.

Based on this knowledge it was concluded that the protein catalysing the addition of the first glycine residue is playing the most important role for cell wall stability and expression of methicillin resistance. Hence it is concluded that this gene and its products provide a promising antimicrobial target and modulation, in particular inhibition of said function will concomitantly impair cell wall stability and antibiotic resistance. However, so far the gene encoding for the protein which is needed for the attachment of the first glycine-residue has not yet been identified.

Recently three additional *femAB* like open reading frames in *S. aureus* have been published. One of them (*fmhB*) seemed to be essential for bacterial survival although proof of its biological function was still pending ³⁾.

Hence the object of the present invention was to identify the gene encoding for the protein which catalyzes the attachment of the first glycine of the staphylococcal interpeptide bridge and to profile its importance for bacterial growth and methicillin resistance.

*fmhB* was chosen as the most promising candidate for the protein responsible for the attachment of the first glycine residue.

In order to prove the above, the xylose regulon of *S. xylosus* ⁴⁾ was introduced into *S. aureus* upstream of *fmhB* thereby allowing for glucose controlled repression of FmhB.

A clear correlation could be demonstrated between *fmhB* repression and accumulation of unsubstituted peptidoglycan monomer precursors, proving the assumption that FmhB is the crucial protein for the attachment of the first glycine residue within the interpeptide bridge during cell wall synthesis.

### MATERIALS AND METHODS

### Strains, Plasmids, and Culture Conditions

All *S. aureus* strains used in this study are derivatives of strain NCTC8325. *S. aureus* RN4220 is a phage-less, restriction negative strain transformable by electroporation. *S. aureus* strain BB255 was used as template for gene amplifications, and the methicillin resistant derivative of BB255, strain BB270 was used for *fmhB* promoter replacement.

The plasmid pCX15 containing the xylose regulatory system of *Staphylocccus carnosus* was obtained from K. P. Wieland, Tübingen.

The *E. coli - S. aureus* shuttle vector pOX7, temperature sensitive (ts) for replication in *S. aureus*, conferring ampicillin resistance to *E. coli*, and erythromycin resistance to *S. aureus*, was obtained from K. Dyke, Oxford

Growth medium was Luria Bertani broth (LB) (Difco, Detroit, Mich.) supplemented with 0.5 % glucose or 0.5 % xylose where indicated.

Transductions in *S. aureus* were made with generalized transducing phage 80α. Transformants and transductants were selected in presence of 20 µg/ml of erythromycin at 30°C. *S. aureus* strains with chromosomally integrated recombinant ts plasmid were propagated in presence of 10 µg/ml of erythromycin at 42°C to maintain plasmid integration.

### Susceptibility tests

The MIC of methicillin was measured by the E-test (AB Biodisk, Solna, Sweden) When determining MICs in presence of glucose, the inoculum was prepared from about 16h cultures grown on plates containing glucose. Growth was read after 24 h incubation at either 35 or 42°C.

### DNA manipulations.

Routine DNA manipulations were done according the protocols of Ausubel et al. and Maniatis et al.. Sequencing was performed with the ABI 310 automated sequencer (Perkin Elmer).

### Isolation of RNA and Northern blots

About 16h cultures of *S. aureus* in LB were diluted 1:50 in LB plus supplements as indicated and grown to mid-log phase (OD₆₀₀ =0.7) or stationary phase (OD₆₀₀ = 2.7). The cells were harvested and processed with a FastRNA isolation kit (Bio 101, Inc., Vista, Calif) containing Trizol reagent (Gibco Life Technologies, Basel, CH) in a FastPrep reciprocating shaker (Bio 101).

Five µg of RNA were separated on a 0.8 % denaturing gel, transferred to a Biodyne A nylon membrane (Pall, Port Washington, NY) using transfer buffer containing 3M NaCl, 8 mM NaOH and 2 mM Na-lauroylsarcosine. Transcripts were detected with a DIG labelled DNA probe covering *fmhB*. DIG labelling was performed as recommended by Boehringer Mannheim, except for the following modifications: maleic acid buffer contained 0.1 M maleic acid, 3 M NaCl, pH 8, and the blocking buffer contained 0.5 % blocking reagent.

### Insertional Replacement of the fmhB Promoter

To place the *fmhB* promoter of *S. aureus* under the control of the xylose regulon of *S. xylosus, xylR* and the *xylA* promoter-operator region located within a 1.7 kb *Hin*dIII-*Bam*H1 fragment in plasmid pCX15 were subcloned into the multicloning site of pUC19.

A 630 bp fragment comprising the ribosome binding site and the 5 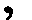 region of *fmhB* (GenBank accession no. AF106849), amplified with the sense primer 1 (5'-GCGGATCCATTGTTAAATAGAAGGAGATATC-3') and reverse primer 2 (5'-GCGGTACCCCCAGTGATTTTCATTAATTC-3') from *S. aureus* BB255, was cloned in the *Bam*HI and *Kpn*I sites downstream of the xylose promoter region.

The resulting fusion of the xylose regulon with the 5 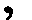 fragment of *fmhB* was sequenced to ensure fidelity of the PCR. The fusion product was excised by *Hin*dIII and *Eco*RI and subcloned into shuttle vector pOX7. The resulting plasmid pSR1 was electroporated into *S. aureus* RN4220 from where it was transduced into strain BB270.

To promote integration of the plasmid pSR1 into the chromosome of BB270, transductants grown at 30°C to stationary phase in LB broth containing 20 µg/ml of erythromycin were diluted 1:100 into fresh LB containing 2.5 µg/ml of erythromycin plus xylose, incubated at 40°C for 8 h, then rediluted 1:100 into fresh LB with 2.5 mg/ml of erythromycin plus xylose and incubated about 16h at 40°C. Appropriate dilutions were spread on LB-agar plates containing 2.5 µg/ml of erythromycin plus xylose and incubated at 42°C. Colonies were checked for plasmid integration by PCR using sense primer 3 (5 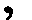 -AAATGAACAATGTGCTATATTACC-3 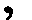 ) and reverse primer 4 (5 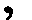 -GCCAGCAAACATTAATAGTGC-3 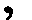 ), as well as sense primer 5 (5 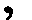 -GCGAATTCTTACACAAGCTCTGAATCGAC-3 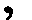 ) and reverse primer 6 (5 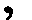 -GCCTGCAGATTTAGCTATTTCGGCATGAAG-3 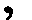 ). In the resulting strain SR18 (*mec, fmhB*::pSR1) chromosomal integration was verified by Southern blot using a probe covering *fmhB* from the start codon to the *Hin*dIII site.

### Isolation of peptidoglycan, preparation and fractionation of muropeptides

Five ml of 16h cultures of strain SR18 or BB270 grown at 42°C were diluted into 500 ml of fresh prewarmed medium, grown aerobically to the exponential growth phase (OD₆₀₀ of 0.7) and harvested by centrifugation (8000 rpm, 15 min, 4°C). The pelleted cells were resuspended in 10 ml of 1 M NaCl and broken with glass beads (0.1 mm), using a cell grinder for 5 min at 4°C. Glass beads were separated off by filtration and washed three times with 30ml of 0.5% sodium dodecyl sulfate (SDS). The collected cell suspension was incubated at 60°C for 30 min to remove noncovalently bound components.

To remove the SDS cell walls were centrifuged three times (8000 rpm, 10 min, 20°C), each time washing the pellet with 100 ml water. Protein A was removed by incubation with 20 ml of 0.2 mg trypsin per ml in 1M Tris-HCl (pH 7.0) for 24 h at 37°C. Samples were three times centrifuged (10000 rpm, 10 min, 4°C), consecutively using 1M Tris/Cl pH7.0, 1M Tris/Cl pH7.0 containing 1M NaCl, and 1M Tris/Cl pH7.0. Finally the probes were three times centrifuged (10000 rpm, 10 min, 4°C). After each centrifugation step the pellet was resuspended in water. The resulting pellet was resuspended in 1 ml of 40% (wt/vol) aqueous hydrofluoric acid and incubated for 18 h at 4°C to remove teichoic acids. Purified murein was finally obtained after submitting the probe to four additional centrifugation (10000 rpm, 10 mm, 4°C) and washing (1 ml water) cycles , and lyophilization. In order to degrade the murein into muropeptides the lyophilized probe (1.3 mg) was dissolved in 0.3 ml of sodium citrate buffer (pH7.0) containing 4 mM MgCl₂ 0.02% sodium azide and 7.5 µg *Streptomyces globisporus* mutanolysin (Sigma), then incubated at 37°C for 16 hours. Samples were adjusted to pH 4.0 with phosphoric acid and incubated at 100°C for 5 min. Immediately after the addition of 175 µl of 1.5 M sodium borate buffer (pH 9.0), 10 mg of solid sodium borohydride was added followed by incubation at room temperature for 15 min to reduce muropeptides to the corresponding muramitol derivatives. Excess borohydride was destroyed by the addition of 20% H₃PO₄. To hydrolyze remaining O-acetyl groups present at the muramic acid residues, samples were adjusted to pH12 with 4 N NaOH and incubated at 37°C for 1.5 h. The muropeptide solution was then adjusted to pH 2.5 with 4 N HCl prior to centrifugation (13,000 x g, 10 min, 4 °C) and filtration (0.22-µm-pore-size filter) to remove insoluble contaminants.

Muropeptides were separated by reversed-phase high-pressure liquid chromatography (HPLC). The HPLC system (waters) consisted of a 600 S controller, a 626 pump, a model 717 autosampler, and a 996 photodiode array detector. Millenium software was used for recording. The column (3-µm Hypersil ODS, 4.0- by 250-mm CompAx-peek; precolumn, 14 by 4.0 mm; Knauer, Berlin, Germany) was eluted at 45°C with a linear gradient beginning with 5% methanol in 50 mM sodium phosphate (pH 2.5) containing 0.0005% sodium azide and ending with 30% methanol in 50 mM sodium phosphate (pH 2.8) during 210 min. The flow-rate was 0.75 ml/min. Muropeptides were detected at 206 nm and identified by amino acid analysis, mass spectrometry and comparison with known control samples.

### Desalting of muropeptides

Selected peaks from a muropeptide separation were collected, chromatographed again under the same conditions and desalted on a Waters 626 HPLC system using a ODS Hypersil column (Knauer, Berlin, Germany) with a particle size of 3 µm and column dimensions of 4x250 mm. Elution was performed at 30°C with a linear gradient beginning with 0.05 % trifluoroacetic acid (TFA) in water and ending with 0.035 % TFA in 30 % CH₃CN in 100 min starting 10 min after injection of the sample. The flow rate was 0.5 ml/min. Using these conditions desalted muropeptides were eluted between 45 and 55 min.

### Amino acid analysis and mass spectrometry

For amino acid analysis, 1 mg of lyophilized murein or the desalted muropeptide were hydrolyzed in 6 N HCl at 166°C for 1 h, dried and subjected to a Biotronic LC 5000 amino acid analyzer. For mass spectrometry the desalted and lyophilized muropeptide was dissolved in 100 µl of 5 % CH₃CN containing 0.035 % TFA. Two µl of the sample were introduced into a Finnigan LCQ mass spectrometer (Thermoquest, Germany) by loop injection. The flow rate of the solvent (0.035 % TFA in 50 % CH₃CN) was 40 µl/min. Data acquisition was performed between 200 and 1800 Da with scan times in the order of 2 to 3 s.

### RESULTS

### Construction of SR18 (mec,fmhB::pSR1)

In order to regulate the expression of *fmhB,* the *fmhB* promoter region in the *S. aureus* chromosome was replaced by the glucose repressible xylose promoter-operator region of *S. xylosus.*

For this purpose a shuttle plasmid, pSR1, temperature sensitive for replication in *S*. *aureus*, containing the xylose regulon of *S. xylosus* fused to the 5 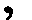 end of the *fmhB* gene was constructed and integrated into the *S. aureus* chromosome by homologous recombination.

This led to replacement of the *fmhB* promoter with the glucose repressible xylose regulon fused to the *fmhB* gene. This promoter replacement was done in methicillin resistant strain BB270 to monitor simultaneously the effects of *fmhB* repression on cell wall composition as well as on the expression of methicillin resistance.

PCR on chromosomal DNA of strain SR18 with primer pair 3/4 produced a 1041 bp band, whereas with primer pair 5/6 no product was detected, indicating chromosomal insertion of pSR1 into the *fmhB* gene.

Additionally Southern blots were performed with a probe covering both sides of the 3 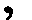 end of the *fmhB* fragment in pSR1. *Hin*dIII digests of chromosomal DNA produced a single 3.1 kb band in BB255 and two bands of 2.6 and 9 kb in SR18. No free plasmid pSR1 was detectable, confirming the correct integration of the construct.

Strain SR18 had to be maintained at 42°C in the presence of erythromycin to prevent excision of the integrated pSR1 plasmid.

The functional proof that the xylose regulon controlled *fmhB* was the slower growth of SR18 in presence of glucose, resulting from repression of *fmhB*.

Northern blot analysis showed that the transcript had a length of about 1600 nt, suggesting that *fmhB* is a monocistronic gene, and that the addition of glucose drastically reduced the *fmhB t*ranscription.

Although strongly repressed by glucose, there was still a considerable leakiness of the *xyl* promoter, which may explain why SR18 continued growth, albeit at a reduced rate, in presence of glucose.

### Cell wall composition of S. aureus SR18

To confirm the role of *fmhB* in cell wall biosynthesis the amino acid composition of the peptidoglycan isolated from strain SR18 grown at 42°C in presence of glucose or xylose, was compared to that of the parental strain BB270 grown under identical conditions.

The amino acid composition of the cell walls of the parent strain BB270 and strain SR18 grown in presence of xylose were in agreement with the well-known composition of the *S. aureus* peptidoglycan; the molar ratio of glutamic acid to glycine was in the range of 1:4 - 1:5.

In contrast, in strain SR18 grown in presence of glucose, a reduction in the cell wall glycine content in relation to glutamic acid was observed.

This allows the conclusion that repression of *fmhB* caused these drastic changes in the amino acid composition of the staphylococcal peptidoglycan and that FmhB is crucial for the normal composition of the cell wall.

### Muropeptide analysis of S. aureus SR18

To characterize the role of FmhB in cell wall biosynthesis in more detail, muropeptides isolated from parent BB270 and mutant SR18 grown in the presence or absence of glucose were analysed.

The HPLC profile derived from the peptidoglycan of the strain BB270 grown at 42°C revealed the typical muropeptide pattern of a wildtype *S. aureus* strain with a high amount of oligomeric muropeptides and the highest peak in the dimeric fraction.

Comparatively no differences in the cell wall composition of the wild type strain upon growth in presence of either glucose or xylose could be observed.

The cell wall *of S. aureus* SR18 grown in presence of xylose revealed the typical bell-shaped muropeptide profile of the HPLC, superimposable to that of strain BB270.

In contrast, a strikingly different muropeptide profile was obtained from *S. aureus* SR18 when grown in presence of glucose.

The amount of oligomeric muropeptides was drastically reduced and the highest peak was found in the monomeric muropeptide fraction, indicating a reduced overall peptidoglycan crosslinkage which was found to be about 65% compared to 80% for the wildtype.

This is mainly due to the drastic increase of the relative amounts of the monomeric muropeptides at the expense of the oligomeric fraction.

A detailed analysis of the monomeric and dimeric muropeptide fraction of *S. aureus* SR18 grown in the presence of glucose revealed a drastic change in cell wall composition.

The monomer pattern was dominated by the non-substituted pentapeptide.

Concomitantly, the amounts of muropeptides modified by one, three or five glycine residues were drastically reduced.

In contrast to the monomeric fraction, the dimer pattern of glucose grown SR18 was found to be similar to that of a wild type strain, although the amounts of these muropeptides were strongly reduced.

The peak corresponding to two crosslinked monomers carrying pentaglycine side-chains was the same as in the wild type or in *S. aureus* SR18 grown in the presence of xylose.

From this result, it can be assumed that the unmodified muropeptide found to a large extend in the monomeric fraction cannot be utilized efficiently for crosslinkage in the peptidoglycan network.

### Structure of the drastically increased monomeric muropeptide obtained in case of fmhB repression

To verify the structure of that muropeptide found in the glucose-repressed strain SR18, it was isolated and subjected to amino acid analysis. The following molar mass ratios in relation to glutamic acid (1.0) were found: alanine, 3.01, and lysine, 1.08. No glycine or other amino acids were detected, consistent with the amino acid composition of an unmodified monomeric muropeptide.

Further mass spectrometry analysis of the whole peptide indicated a molecular mass of 968.4 Da whitch is the expected mass for the unmodified muropeptide carrying noyy (10000 rpm, 10 min, 4°C) (10000 rpm, 10 min, 4°C) (10000 rpm, 10 min 4°C) ycine interpeptide bridge.

When combining the results of amino acid composition and mass spectrometry data the proposed structure GlcNAc-MurNAc-AQKAA for the increased monomeric muropeptide found in the *fmhB* repressed strain was confirmed.

### Susceptibility to methicillin

Methicillin resistance in *S. aureus* is due to the acquisition of the foreign PBP2 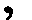 and can only be expressed when the pentaglycine interpeptide has the correct length. This resistance is abolished in *femAB* null mutants which produce a peptidoglycan precursor with only one glycine residue. A similar effect was expected to occur in SR18 after *fmhB* repression.

Since this strain had to be maintained at 42°C one must take into account the temperature dependence of methicillin resistance. At 35°C the methicillin resistant parent BB270 had an MIC of methicillin of 16 µg/ml which was reduced to 1.5 µg/ml at 42°C.

Strain SR18 had a similar MIC of methicillin of 1 µg/ml at 42°C, and in presence of glucose the MIC was reduced to 0.19 µg/ml.

This reduction of methicillin resistance in strain SR18 is unambiguously due to the repression of *fmhB*, because addition of glucose to the medium had no significant effect on the methicillin MIC in the parent strain BB270 at either temperature.

In summary, we have shown that repression of *fmhB* causes a drastic increase in muropeptides lacking the glycine interpeptides and glycine at all.

The structure of the accumulating unsubstituted muropeptide, was confirmed by mass spectrometry, and it was therefore proved that FmhB is the protein which is essential for the addition of the first glycine residue into the staphylococcal pentaglycine interpeptide.

Thus *fmhB*, its homologues and their corresponding gene products provide a novel antimicrobial target for modulating or in particular inhibiting compounds which can be used in antibiotic therapy of staphylococcal or other microbial infections.
1) K. Ehlert, Current Pharmaceutical Design, 1999, 5, 45-55
2) Song, M. D., Wachi, M., Doi, M., Ishino, F. & Matsuhashi, M. (1987) FEBS Lett 221, 167-171.
3) Tschierske, M., Mori, C., Rohrer, S., Ehlert, K., Shaw, K. J. & Berger-Bächi, B. (1999) FEMS Microbiol Lett 171, 97-102.
4) Wieland, K.-P., Wieland, B. & Götz, F. (1995) Gene 158, 91-96.

## Claims

1. Assay for the determination of the activity of FmhB and its homologues.

2. Assay according to claim 1 wherein the binding of the first glycine to a murein precursor is determined

3. Assay according to any of claims 1 and 2 wherein an unmodified murein precursor is used.

4. Assay wherein the indicator system is based on a reaction mediated by a lysostaphin endopeptidase thereby detecting the activity of FmhB, FemAB, and/or their homologues.

5. Use of FmhB in the screening for antimicrobial compounds

6. Compounds capable to bind to FmhB

7. Compounds capable to modulate the activity of FmhB

8. Compounds capable to inhibit the activity of FmhB

9. Pharmaceutical composition comprising a compound according to anyone of claims 6 to 8.

10. Pharmaceutical composition or combination comprising a compound according to any of claims 6 to 8 and an antibiotic.
